# EUROPEAN PATENT APPLICATION

(11) **EP 3 653 220 A2**
(43) Date of publication of application: **20.05.2020**
(21) Application number: 18832897.5
(22) Date of filing: 06.07.2018
(51) Int. Cl.: A61K 36/288, A61K 36/53, A23L 33/105

(54) **COMPOSITION FOR PROTECTING LIVER COMPRISING DANDELION EXTRACT AND LEMON BALM EXTRACT**

(30) Priority: 12.07.2017 KR 20170088605
(71) Applicant: Phytonutrimedi Co., Ltd., Jeonju-si, Jeollabuk-do 54969 (KR)
(72) Inventor: SO, Byoung Ok, Gwangju 61704 (KR); JUNG, Su Jin, Jeonju-si Jeollabuk-do 54899 (KR)
(74) Representative: Wittmer, Maximilian
(86) International application number: PCT/KR2018/007681
(87) International publication number: WO 2019/013495

(57) **Abstract**

The present invention can effectively protect a liver from oxidative stress caused by liver damage by significantly reducing the aspartate aminotransferase (AST) and alanine aminotransferase (ALT) values, which are widely known as indicators of liver damage when applied to liver toxicity animal models. The present invention relates to a composition for protecting a liver comprising a dandelion extract and a lemon balm extract as active ingredients that can be usefully used for preventing, ameliorating or treating liver damage, wherein the composition is characterized by comprising a dandelion extract and a lemon balm extract as active ingredients.

## Description

The present invention relates to a composition for protecting a liver, comprising a dandelion extract and a lemon balm extract and more particularly to a composition for protecting a liver, comprising a dandelion extract and a lemon balm extract as effective ingredients, which may effectively protect a liver from oxidative stress caused by liver damage by significantly reducing the aspartate aminotransferase (AST) and alanine aminotransferase (ALT) values, which are widely known as indicators of liver damage when applied to liver toxicity animal models, and may be usefully used for preventing, ameliorating or treating the liver damage.

A liver is an important organ which performs various physiological activities such as protein synthesis, blood sugar balance and detoxification and also takes charge of metabolizing various nutrients. In general, liver damage occurs when being exposed to a large amount of toxic ingredients derived from the environment, and may be accompanied by a wide scope of pathological changes ranging from a temporarily slight rise in liver enzyme levels to life-threatening hepatic fibrosis, hepatic cirrhosis and hepatocarcinoma. Until now, various causes have been pointed out as a main culprit of liver damage, among which oxidative stress and inflammation have been accepted as the most important causes. As liver diseases occur with the depletion of an antioxidant defense system caused by a chemical organic solvent, i.e., CCl₄, an experimental animal with CCl₄-induced liver damage has been now frequently used in developing a liver protective agent and conducting a pathogenetic study on the liver diseases (Yang BY, Zhang XY, Guan SW, Hua ZC. Protective effect of procyanidin B2 against CCl4-induced acute liver injury in mice. Molecules. 2015;20:12250-65.; Zou J, Qi F, Ye L, Yao S. Protective Role of grape seed proanthocyanidins against CCl4 induced acute liver injury in mice. Med Sci Monit. 2016;22:880-9).

In the experimental animal, the CCl₄-induced liver damage shows various pathological states depending on an amount of exposure and a period of time. In other words, it is known that a low dose of CCl₄ causes temporary disorders such as a loss of Ca²⁺ ion regulatory capacity, a disorder of fat metabolism and a liberation of inflammatory cytokines, but a long-term exposure to CCl₄ leads to a disorder of fatty acid metabolism and thus causes liver fibrosis, liver cirrhosis and liver cancer (Cui Y, Yang X, Lu X, Chen J, Zhao Y. Protective effects of polyphenols-enriched extract from Huangshan Maofeng green tea against CCl4-induced liver injury in mice. Chem Biol Interact. 2014;220:75-83). Now, CCl₄-induced liver toxicity is known to form unstable trichloromethyl and trichloromethyl peroxyl radicals which may bind with protein or lipid through a reductive dehalogenation reaction metabolized by a hepatic metabolic enzyme, i.e., cytochrome P-450 and thus causes lipid peroxidation and damage to liver cells (Cheng N, Ren N, Gao H, Lei X, Zheng J, Cao W. Antioxidant and hepatoprotective effects of Schisandra chinensis pollen extract on CCl4-induced acute liver damage in mice. Food Chem Toxicol. 2013;55:234-40). Thus, an oxidative stress caused by the radicals produced by CCl₄ is known as a key mechanism of liver damage. Such radicals cause damage to the membrane of liver cells and thus bring about a rise in liver enzyme levels. Further, inflammation is known as another mechanism of aggravating the liver damage due to CCl₄ (Ebaid H, Bashandy SA, Alhazza IM, Rady A, El-Shehry S. Folic acid and melatonin ameliorate carbon tetrachloride-induced hepatic injury, oxidative stress and inflammation in rats. Nutr Metab (Lond). 2013 3;10:20.; Yang BY, Zhang XY, Guan SW, Hua ZC. Protective effect of procyanidin B2 against CCl4-induced acute liver injury in mice. Molecules. 2015;20:12250-65). Thus, a medicinal efficacy of candidate substances in an experimental animal model with CCl₄-induced liver damage has been evaluated mainly through a histopathological examination based on anti-inflammatory and antioxidant effects (Ferreira EA, Gris EF, Felipe KB, Correia JF, Cargnin-Ferreira E, Wilhelm Filho D, Pedrosa RC. Potent hepatoprotective effect in CCl(4)-induced hepatic injury in mice of phloroacetophenone from Myrcia multiflora. Libyan J Med. 2010;5.; Wang DH, Wang YN, Ge JY, Liu HY, Zhang HJ, Qi Y, Liu ZH, Cui XL. Role of activin A in carbon tetrachloride-induced acute liver injury. World J Gastroenterol. 2013;19:3802-9).

The development of liver protective drugs has been continuously conducted. However, with a demand for developing drugs with a less side effect and a powerful liver protective effect, an exploration into the liver protective drugs using natural products has been still in active progress. Out of those products, silymarin, which is a flavonoid derived from thistle (milk thistle, Silibum marianum), is a representative liver protective drug derived from natural products, showing a liver protective effect based on a powerful antioxidant effect (Wellington K, Jarvis B. Silymarin: a review of its clinical properties in the management of hepatic disorders. BioDrugs. 2001;15:465-89). In an experimental animal model with CCl₄-induced liver damage, silymarin is also known to show a liver protective effect based on a powerful antioxidant effect and thus has been used as an important control drug in developing various liver protective drugs (Ferreira EA, Gris EF, Felipe KB, Correia JF, Cargnin-Ferreira E, Wilhelm Filho D, Pedrosa RC. Potent hepatoprotective effect in CCl(4)-induced hepatic injury in mice of phloroacetophenone from Myrcia multiflora. Libyan J Med. 2010;5.; Jain NK, Lodhi S, Jain A, Nahata A, Singhai AK. Effects of Phyllanthus acidus (L.) Skeels fruit on carbon tetrachloride-induced acute oxidative damage in livers of rats and mice. Zhong Xi Yi Jie He Xue Bao. 2011;9:49-56.; Wang R, Feng X, Zhu K, Zhao X, Suo H. Preventive activity of banana peel polyphenols on CCl(4)-induced experimental hepatic injury in Kunming mice. Exp Ther Med. 2016;11:1947-54). In this context, silymarin has been selected as a control drug in the present experiment, too.

The present invention can effectively protect a liver from oxidative stress caused by liver damage by significantly reducing the aspartate aminotransferase (AST) and alanine aminotransferase (ALT) values, which are widely known as indicators of liver damage when applied to liver toxicity animal models. An objective of the present invention is to provide a composition for protecting a liver, comprising a dandelion extract and a lemon balm extract as effective ingredients that can be usefully used for preventing, ameliorating or treating liver damage.

According to the present invention, a composition having an efficacy of improving liver functions is characterized by containing a dandelion extract and a lemon balm extract as effective ingredients.

The dandelion extract may be extracted from dandelion roots, whole dandelion plants, preferably dandelion leaves with a polar solvent selected from the group consisting of water, methanol, ethanol and a mixture of at least two thereof.

The lemon balm extract may be extracted from whole lemon balm plants, preferably lemon balm leaves with a polar solvent selected from the group consisting of water, methanol, ethanol and a mixture of at least two thereof.

The composition may contain the dandelion extract and the lemon balm extract at a weight ratio of 1:8 to 8:1, preferably 1:1 to 4.

The composition may be a drug or a food containing the composition as an effective ingredient.

As such, the present invention has an effect of protecting a liver from oxidative stress caused by liver damage by significantly reducing the aspartate aminotransferase (AST) and alanine aminotransferase (ALT) values, which are widely known as indicators of liver damage when applied to liver toxicity animal models.
Fig. 1 is a graph of showing a change in weights after acute liver damage induced by CCl₄ administration after administering a test substance.
Figs. 2 and 3 are the graphs of showing findings in gross liver autopsy and a change in weights after acute liver damage induced by CCl₄ administration after administering a test substance.
Fig. 4 is a graph of showing a change in AST and ALT contents in blood after acute liver damage induced by CCl₄ administration after administering a test substance.
Fig. 5 is a graph of showing a histopathological change after acute liver damage induced by CCl₄ administration after administering a test substance.

The present invention, in a desired aspect, provides a composition having an efficacy of improving liver functions, characterized by containing a dandelion extract and a lemon balm extract as effective ingredients, and also provides a drug having an efficacy of improving liver functions and a food having an efficacy of improving liver functions, which contain such composition as an effective ingredient.

Hereinafter, the specific exemplary embodiments of the present invention will be described in more detail with reference to the accompanying drawings.

Dandelion (*Taraxacum officinale*), which is a plant belonging to the chrysanthemum family (*Asteraceae*), is well-known to have an effect on reduction in blood glucose levels, anti-inflammation, anticancer and cardio-protection, and thus has been traditionally used for various liver diseases, gallbladder disorder, digestive disorder, rheumatoid arthritis, etc. (Davaatseren M, Hur HJ, Yang HJ, Hwang JT, Park JH, Kim HJ, Kim MJ, Kwon DY, Sung MJ. Taraxacum official (dandelion) leaf extract alleviates high-fat diet-induced nonalcoholic fatty liver. Food Chem Toxicol. 2013;58:30-6). So far, taraxin, taraxerol, caffeic acid, flavoxanthin, taraxacin, taraxasterol, choline, inulin, pectin, desacetylmatricarin, achillin and leucodin have been identified as main active ingredients of dandelion (Gu JH, Kim SR, Lee JW, Park MY, Choi JY, Kim JD. Mouse single oral dose toxicity test of Taraxaci Herba aqueous extracts. Korean J. Oriental Physiology & Pathology. 2011;25:650-7). In particular, a liver protective effect of a dandelion extract has been experimentally identified through a CCl₄-induced acute liver damage model, a high-fat diet non-alcoholic fatty liver model, a streptozotocin-induced diabetic hepatopathy model, etc. (Cho SY, Park JY, Park EM, Choi MS, Lee MK, Jeon SM, Jang MK, Kim MJ, Park YB. Alternation of hepatic antioxidant enzyme activities and lipid profile in streptozotocin-induced diabetic rats by supplementation of dandelion water extract. Clin Chim Acta. 2002;317:109-17), and a valid effect on CCl₄-induced hepatic fibrosist has been well-known, too (DomitroviR, Jakovac H, RomiZ, RaheliD, TadiZ. Antifibrotic activity of Taraxacum officinale root in carbon tetrachloride-induced liver damage in mice. J Ethnopharmacol. 2010;130:569-77).

In addition, lemon balm (*Melissa officinalis*), which is a plant belonging to the *Labiatae* family, is known to contain various active flavonoids (cynaroside, cosmosin, rhamnocitrin and isoquercitrin), terpene and triterpene acids (ursolic acid and oleanolic acid) (Herodez SS, Hadolin M, Skerget M, Knez Z. Solvent extraction study of antioxidants from balm (*Melissa officinalis* L.) leaves. Food Chemistry. 200;80:275-82). Active ingredients such as essential oil, caffeic acid and rosmarinic acid, monoterpene, sesquiterpene, phenolic substances, tannin and the like are separated and extracted from lemon balm, too. So far, a lemon balm extract has been reported to have the anti-septic (Ulbricht C, Brendler T, Gruenwald J, Kligler B, Keifer D, Abrams TR, Woods J, Boon H, Kirkwood CD, Hackman DA, Basch E, Lafferty HJ; Natural Standard Research Collaboration. Lemon balm (Melissa officinalis L.): an evidence-based systematic review by the Natural Standard Research Collaboration. J Herb Pharmacother. 2005;5:71-114), anti-virus, antibacterial and antioxidant activities, and the liver protective effect of the lemon balm extract has been also well-known in a hyperlipidemia rat model and a high cholesterol rat model (Bolkent S, Yanardag R, Karabulut-Bulan O, Yesilyaprak B. Protective role of Melissa officinalis L. extract on liver of hyperlipidemic rats: a morphological and biochemical study. J Ethnopharmacol. 2005;99:391-8, Ali Zarei1, Saeed Changizi Ashtiyani, Soheila Taheri, Fateme Rasekh Comparison between effects of different doses of officinalisatorvastatin on the activity of liver enzymes in hypercholesterolemia rats. AJP 2014; 4(1) 15-23).

The dandelion extract is prepared by carrying out an extraction from whole dandelion plants, preferably dandelion leaves with a polar solvent, preferably water, selected from the group consisting of water, methanol, ethanol and a mixture of at least two thereof, and spray-drying the resulting extract into powder.

The lemon balm extract is prepared by carrying out an extraction from whole lemon balm plants, preferably lemon balm leaves with a polar solvent, preferably water, selected from the group consisting of water, methanol, ethanol and a mixture of at least two thereof, and spray-drying the resulting extract into powder.

Hereinafter, the exemplary examples and comparative examples of the present invention will be described.

The examples described below are provided only for illustrative purposes, but are not to be construed to limit the scope of the present invention.

### Preparation Example 1: Preparation of lemon balm leaf extract powder (LB)

Lemon balm leaves were washed and dried in the shade, after which 30 kg of the dried lemon balm leaves were accurately selected, then washed cleanly, and then put into an extractor at a ratio of original stuff: solvent = 3:20 (kg:kg) to carry out an extraction at 121°C and 0.15 Mpa for six hours. This process was repeated to perform the extraction twice. As the solvent, water or 70% ethanol (ethanol or alcohol) was used. A solid content was removed from the resulting extract through filtration, after which the resulting filtrate was concentrated under reduced pressure to obtain a concentrate of 25 brix (±5 brix). The resulting concentrate and dextrin were mixed at a ratio of 9:1 (kg:kg) and pulverized into powder with a spray dryer.

### Preparation Example 2: Preparation of dandelion leaf extract powder (DL)

Instead of lemon balm leaves, dandelion leaves were used. Those leaves were weighed and collected in the exact amount of 30 kg, then washed clearly, and then put into an extractor at a ratio of original stuff: solvent = 3 : 20 (kg : kg) to carry out an extraction at 121°C and 0.15 Mpa for six hours. This process was repeated to perform the extraction twice. As the solvent, water or 70% ethanol (ethanol or alcohol) was used. A solid content was removed from the resulting extract through filtration, after which the resulting filtrate was concentrated under reduced pressure to obtain a concentrate of 25 brix (±5 brix). The resulting concentrate and dextrin were mixed at a ratio of 9:1 (kg:kg) and pulverized into powder with a spray dryer.

### Outline of Experiment:

LB and DL single compositions and nine species of LB and DL mixed compositions (at a ratio of LB:DL = 1:1, 1:2, 1:4, 1:6, 1:8, 2:1, 4:1, 6:1 and 8:1, g/g) were respectively dissolved in sterile distilled water, and then orally administered once a day for seven days at a dose of 10 ml/g (200 mg/kg), after which CCl₄ was intraperitoneally administered once at 0.5 ml/kg in one hour after a last seventh oral administration to induce an acute liver damage. In 24 hours after CCl₄ administration, observation was made along with findings in gross liver autopsy, liver weights, AST and ALT contents in blood, lipid peroxidation of liver antioxidant defense system, GSH content and histopathological change in liver tissues with SOD and CAT activities, and a change in weights and weight gains was also observed for the whole experimental period of seven days. Histopathologically, a degree of liver damage was evaluated based on a change in HAI grading scores (Ishak K, Baptista A, Bianchi L, Callea F, De Groote J, Gudat F, Denk H, Desmet V, Korb G, MacSween RN, Phillips MJ, Portmannl BG, Paulsen H, Scheuer PJ, Schmid M, Thaler H. Histological grading and staging of chronic hepatitis. J Hepatol. 1995;22:696-9), a rate of degenerated regions in liver parenchyma (%/mm²), the number of degenerated liver cells (cells/1000 liver cells) and the number of infiltrating inflammatory cells (cells/mm²) (Ki SH, Yang JH, Ku SK, Kim SC, Kim YW, Cho IJ. Red ginseng extract protects against carbon tetrachloride-induced liver fibrosis. J Ginseng Res. 2013;37:45-53). In the present experiment, in accordance with a prior report (Kang SJ, Choi BR, Kim SH, Yi HY, Park HR, Song CH, Ku SK, Lee YJ. Selection of the optimal herbal compositions of red clover and pomegranate according to their protective effect against climacteric symptoms in ovariectomized mice. Nutrients. 2016;8. pii: E447), an LB:DL mixed composition was considered to simultaneously show a synergy effect with a statistically significant rise in a medicinal efficacy (p<0.01 or p<0.05) compared to each of LB and DL single compositions. Further, silymarin, which is a liver protective agent derived from natural products and has been already well-known to have a liver protective effect based on its antioxidant effect, was used as a control drug. Accordingly, comparison and evaluation were made in such a way that a group orally dosed with silymarin 100 mg/kg was used as a control drug group (Jain NK, Lodhi S, Jain A, Nahata A, Singhai AK. Effects of Phyllanthus acidus (L.) Skeels fruit on carbon tetrachloride-induced acute oxidative damage in livers of rats and mice. Zhong Xi Yi Jie He Xue Bao. 2011;9:49-56).

A total of 126 individual SPF/VAF Outbred CrljOri:CD1[ICR] male mice (OrientBio, Seungnam, Korea) were acclimated for eight days, then screened and divided into each group of eight individuals based on weights (35.07±1.62 g on average and 31.30 to 39.30 g) measured one day before administering an experimental substance, and then a total of 14 groups were used in the experiment. All the experimental animals were fasted (with a free access to drinking water) for 18 hours before starting the administration of experimental substances and before a final day of autopsy.

### Group separation (a total of 14 groups with 8 individuals per group)

- Normal vehicle control group: Normal vehicle control group dosed with olive oil after administration of sterile distilled water
- CCl₄ control group: Vehicle control group with acute liver damage induced by administration of CCl₄ 0.5 ml/kg after administration of sterile distilled water
- Control drug group dosed with silymarin 100 mg/kg and CCl₄ 0.5 ml/kg
- Experimental group dosed with LB single composition 200 mg/kg and CCl₄ 0.5 ml/kg
- Experimental group dosed with DL single composition 200 mg(kg and CCl₄ 0.5 ml/kg
- Experimental group dosed with mixed composition of LB:DL = 1:1, 1:2, 1:4, 1:6, 1:8, 2:1, 4:1, 6:1 and 8:1 in a total amount of 200 mg/kg and CCl₄ 0.5 ml/kg (9 species)

**Administration of experimental substance:** An adequate amount of LB and DL was dissolved in sterile distilled water and then orally administered once daily for seven days at a dose of 10 ml/kg. In other words, each of LB and DL single compositions was dissolved in sterile distilled water at a concentration of 20 mg/ml and then orally administered with a 1 ml syringe attached with a metal probe at a dose of 10 ml/kg (200 mg/kg). With regard to the mixed composition of LB:DL = 1:1, 1:2, 1:4, 1:6, 1:8, 2:1, 4:1, 6:1 and 8:1, each of LB and DL was dissolved in 10 ml of distilled water at a ratio of 100:100, 66:134, 40:160, 28:172, 22:178, 134:66, 160:40, 172:28 and 178:22 (mg:mg) and orally administered at a dose of 10 ml/kg (in a total amount of 200 mg/kg), too. Silymarin (Sigma-Aldrich, St. Louise, MO, USA) was also dissolved in sterile distilled water at a concentration of 10 mg/ml and orally administered once daily for seven days at a dose of 10 ml/kg (100 mg/kg). With regard to normal vehicle and CCl₄ control groups, instead of the experimental substances (LB and DL single compositions, 9 species of LB:DL mixed composition or silymarin), only the same dose of vehicle was orally administered by the same method in order to make the same correction and apply the same dosing stress. An administered dose of LB and DL single compositions used in the present experiment was estimated at 200 mg/kg based on the prior experiments for evaluating a liver protective effect of the dandelion extract in various experimental animal models (Cho SY, Park JY, Park EM, Choi MS, Lee MK, Jeon SM, Jang MK, Kim MJ, Park YB. Alternation of hepatic antioxidant enzyme activities and lipid profile in streptozotocin-induced diabetic rats by supplementation of dandelion water extract. Clin Chim Acta. 2002;317:109-17) and a liver protective effect of the lemon balm extract in hyperlipidemia rats (Bolkent S, Yanardag R, Karabulut-Bulan O, Yesilyaprak B. Protective role of Melissa officinalis L. extract on liver of hyperlipidemic rats: a morphological and biochemical study. J Ethnopharmacol. 2005;99:391-8, and Ali Zarei1, Saeed Changizi Ashtiyani, Soheila Taheri, Fateme Rasekh Comparison between effects of different doses ofofficinalisatorvastatin on the activity of liver enzymes in hypercholesterolemia rats. AJP 2014; 4(1) 15-23) as well as the study conducted by the present inventors, etc., for exploring a medicinal efficacy using a prior mouse model with CCl₄-induced acute liver damage. To compare a medicinal efficacy with the single compositions, an administered dose of the nine species of LB:DL mixed composition was also set at a total amount of 200 mg/kg; and an administered dose of silymarin was also set at 100 mg/kg as shown in the following table 1 based on the prior document (Jain NK, Lodhi S, Jain A, Nahata A, Singhai AK. Effects of Phyllanthus acidus (L.) Skeels fruit on carbon tetrachloride-induced acute oxidative damage in livers of rats and mice. Zhong Xi Yi Jie He Xue Bao. 2011 ;9:49-56).

**Table 1**

| Group | Animal | Test Substance (mg/kg) | Animal Number |
|---|---|---|---|
| Control group | Normal mouse | Distilled water and olive oil | M01-M08 |
| | CCl₄ mouse | Distilled water and CCl₄ 0.5 ml/kg | M09-M16 |
| Comparative group | CCl₄ mouse | Silymarin 100 mg/kg and CCl₄ 0.5 ml/kg | M17-M24 |
| | CCl₄ mouse | LB single 200 mg/kg and CCl₄ 0.5 ml/kg | M25-M32 |
| | CCl₄ mouse | DL single 200 mg/kg and CCl₄ 0.5 ml/kg | M33-M40 |
| Experimental group | CCl₄ mouse | LB:DL = 1:1 200 mg/kg and CCl₄ 0.5 ml/kg | M41-M48 |
| | CCl₄ mouse | LB:DL = 1:2 200 mg/kg and CCl₄ 0.5 ml/kg | M49-M56 |
| | CCl₄ mouse | LB:DL = 1:4 200 mg/kg and CCl₄ 0.5 ml/kg | M57-M64 |
| | CCl₄ mouse | LB:DL = 1:6 200 mg/kg and CCl₄ 0.5 ml/kg | M65-M72 |
| | CCl₄ mouse | LB:DL = 1:8 200 mg/kg and CCl₄ 0.5 ml/kg | M73-M80 |
| | CCl₄ mouse | LB:DL = 2:1 200 mg/kg and CCl₄ 0.5 ml/kg | M81-M88 |
| | CCl₄ mouse | LB:DL = 4:1 200 mg/kg and CCl₄ 0.5 ml/kg | M89-M96 |
| | CCl₄ mouse | LB:DL = 6:1 200 mg/kg and CCl₄ 0.5 ml/kg | M97-M104 |
| | CCl₄ mouse ml/kg | LB:DL = 8:1 200 mg/kg and CCl₄ 0.5 ml/kg | M105-M112 |
| CCl₄ = Carbon tetrachloride; LB = Lemon balm leaf extract; DL = Dandelion leaf extract; LB:DL = Mixture of lemon balm leaf extract and dandelion leaf extract | | | |

**Induction of acute liver damage:** In one hour after administering silymarin 100 mg/kg or carrying out the last seventh administration of the five species of natural product extract, CCl₄ (Sigma-Aldrich, St Louise, MO, USA) was diluted in olive oil (Sigma-Aldrich, St Louise, MO, USA) at 1:19 v/v (5%), and intraperitoneally administered once at a dose of 10 ml/kg (0.5 ml/kg of CCl₄ itself) with a 1 ml syringe attached with a 26 G needle to induce an acute liver damage. Meanwhile, the normal vehicle control group was dosed with the same dose of olive oil by means of the same method instead of CCl₄ mixed liquid. **Observation items:** Weights and weight gains, findings in gross liver autopsy, liver weights, AST and ALT contents in blood, liver antioxidant defense system, and histopathological changes in liver tissues

**Liver antioxidant defense system:** Lipid peroxidation (MDA content), GSH content, SOD and CAT activities

**Histopathological changes:** HAI (histological activity index) grading scores (table 2), a ratio of degenerated regions in liver parenchyma (%/mm²), the number of degenerated liver cells (cells/1000 liver cells) and the number of infiltrating inflammatory cells (cells/mm²)

**Table 2**

| A. Confluent necrosis | |
|---|---|
| Not present | 0 |
| Local confluent necrosis | 1 |
| Zone 3 necrosis in some regions | 2 |
| Zone 3 necrosis in most regions | 3 |
| Zone 3 necrosis + sporadic portal-centered cross-linkage | 4 |
| Zone 3 necrosis + multiple portal-centered cross-linkage | 5 |
| Panacinar necrosis | 6 |

| B. Local bacteriophagic necrosis, apoptosis and local inflammation n | |
|---|---|
| Not present | 0 |
| 1 or less per 10X magnification | 1 |
| 2 to 4 per 10X magnification | 2 |
| 5 to 10 per 10X magnification | 3 |
| More than 10 per 10X magnification | 4 |
| HAI = Histological activity index; HAI grading score = A=B | |

### 1. Change in weights

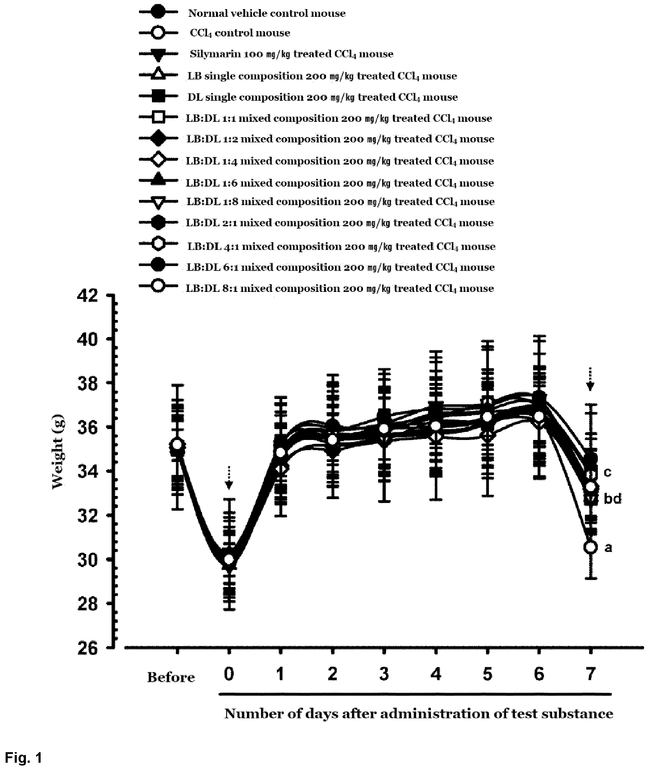

The CCl₄ control group showed a significant decrease in weights (p<0.01) compared to the normal vehicle control group and such decrease was identified exclusively on a final day of sacrifice in 24 hours after CCl₄ administration. In this regard, the former group also showed a significant decrease in weight gains (p<0.01) for seven days compared to the normal vehicle control group. Meanwhile, in all the groups dosed with the experimental substances including silymarin 100 mg/kg, a significant increase in weights (p<0.01 or p<0.05) was identified on a final day of sacrifice in 24 hours after CCl₄ administration compared to the CCl₄ control group, and a significant increase in weight gains (p<0.01) for seven days was also shown compared to the CCl₄ control group. In particular, in the group dosed with the mixed compositions of LB:DL = 2:1, 4:1 and 1:1, a significant increase in weight gains (p<0.01 or p<0.05) was identified in the same order compared to the group dosed with each of LB and DL single compositions (Table 3 and Fig. 1).

**Table 3**

| Group | | Weights at the following points of time | | | Weight gains [B-A] |
|---|---|---|---|---|---|
| | | 1^{st} day after test substance administration [A]* | Last 7^{th} day after test substance administration | In 24 hours after CCl₄ treatment [B]* | |
| Control group | (Normal | 30.08±1.15 | 37.35±0.93 | 34.54±0.93 | 4.46±1.50 |
| | CCl₄ | 29.95±1.31 | 36.49±1.76 | 30.54±1.40^{a} | 0.59±0.38^{e} |
| Comparative group | Silymarin | 30.30±1.82 | 37.29±2.62 | 32.96±1.73^{c} | 2.66±0.77^{eg} |
| | LB single | 29.74±1.49 | 36.40±2.04 | 32.80±1.46^{bd} | 3.06±0.52^{fg} |
| | DL single | 30.00±1.48 | 36.41±1.86 | 32.73±1.37^{bd} | 2.73±0.71^{fg} |
| Experimental group [LB:DL] (g/g) | 1:1 | 30.21±2.49 | 36.90±3.23 | 33.96±2.67^{c} | 3.75±0.62^{ghj} |
| | 1:2 | 30.21±1.69 | 36.21±1.80 | 33.00±1.64^{c} | 3.09±0.88^{fg} |
| | 1:4 | 30.15±1.76 | 36.21±1.96 | 33.19±1.80^{c} | 3.04±0.78^{fg} |
| | 1:6 | 30.24±1.00 | 36.75±1.94 | 33.23±1.43^{c} | 2.99±0.72^{fg} |
| | 1:8 | 29.70±1.00 | 36.69±1.57 | 32.73±1.40^{bd} | 3.03±0.49^{fg} |
| | 2:1 | 29.91±1.82 | 36.54±2.79 | 34.09±2.95^{c} | 4.18±1.21^{ghi} |
| | 4:1 | 29.88±1.42 | 36.99±1.63 | 33.83±1.87^{c} | 3.95±0.77^{ghi} |
| | 6:1 | 30.00±1.11 | 37.00±1.74 | 33.29±1.45^{c} | 3.29±0.67^{g} |
| | 8:1 | 29.99±0.74 | 36.48±1.39 | 33.26±1.37^{c} | 3.28±1.16^{g} |
| The values are indicated as mean±SD of eight individual mice (unit: g). All the animals were fasted all night (for about 18 hours with free access to drinking water). ^{a}p<0.01 and ^{b}p<0.05 compared to the normal control according to an LSD test; ^{c}p<0.01 and ^{d}p<0.05 compared to the CCl₄ control according to the LSD test; ^{e}p<0.01 and ^{f}p<0.05 compared to the normal control according to the MW test; ^{g}p<0.01 compared to the CCl₄ control according to the MW test; ^{h}p<0.05 compared to the LB single composition according to the MW test; and ⁱp<0.01 and ^{j}p<0.05 compared to the DL single composition according to the MW test. LSD = least-significant differences multi-comparison (added with a need for explanation about the abbreviation), and MW= Mann-Whitney U. | | | | | |

With regard to the weight gains for the whole experimental period of seven days, the CCl₄ control group showed a change of -86.83% compared to the normal vehicle control group. However, the groups dosed with silymarin 100 mg/kg, LB and DL single compositions 200 mg/kg, and the mixed compositions of LB:DL = 1:1, 1:2, 1:4, 1:6, 1:8, 2:1, 4:1, 6:1 and 8:1 200 mg/kg showed a change of 353.19, 421.28, 363.83, 538.30, 425.53, 417.02, 408.51, 414.89, 610.64, 572.34, 459.57 and 457.45% compared to the CCl₄ control group, respectively.

### 2. Findings in gross liver autopsy and change in weights

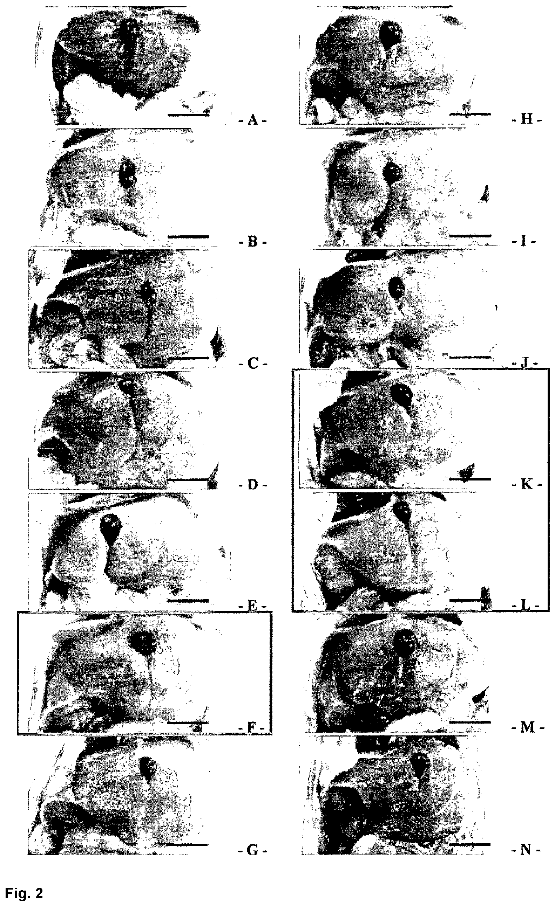

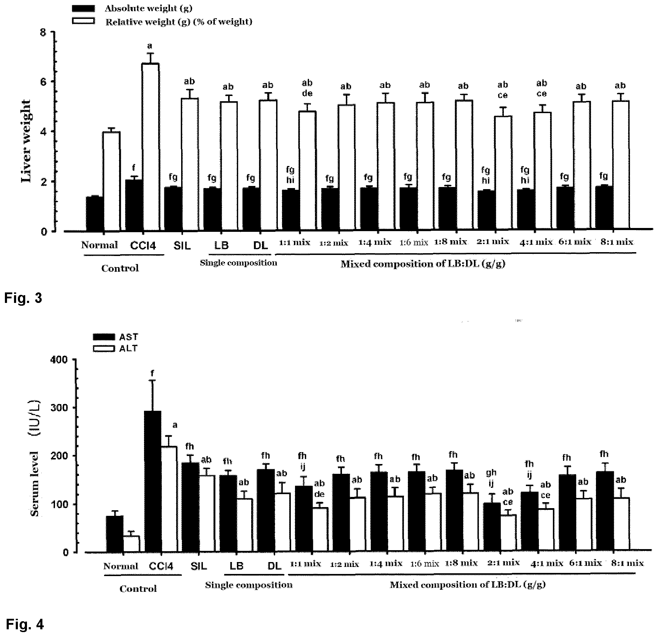

The CCl₄ control group shows findings of hepatomegaly accompanied by a remarkable nodule formation compared to the normal vehicle control group, and thus a significant increase in absolute and relative liver weights (p<0.01) was identified respectively compared to the normal vehicle control group. However, all the groups dosed with experimental substances including the LB single composition 200 mg/kg showed a significant decrease in absolute and relative liver weights (p<0.01) and a remarkable decrease in findings of hepatomegaly accompanied by a nodule formation compared to the CCl₄ control group. In particular, in the groups dosed with the mixed compositions of LB:DL = 2:1, 4:1 and 1:1, a significant decrease in absolute and relative liver weights (p<0.01 or p<0.05) and a remarkable decrease in findings of hepatomegaly accompanied by a nodule formation were identified in the same order compared to the group dosed with each of LB and DL single compositions (Figs. 2 and 3).

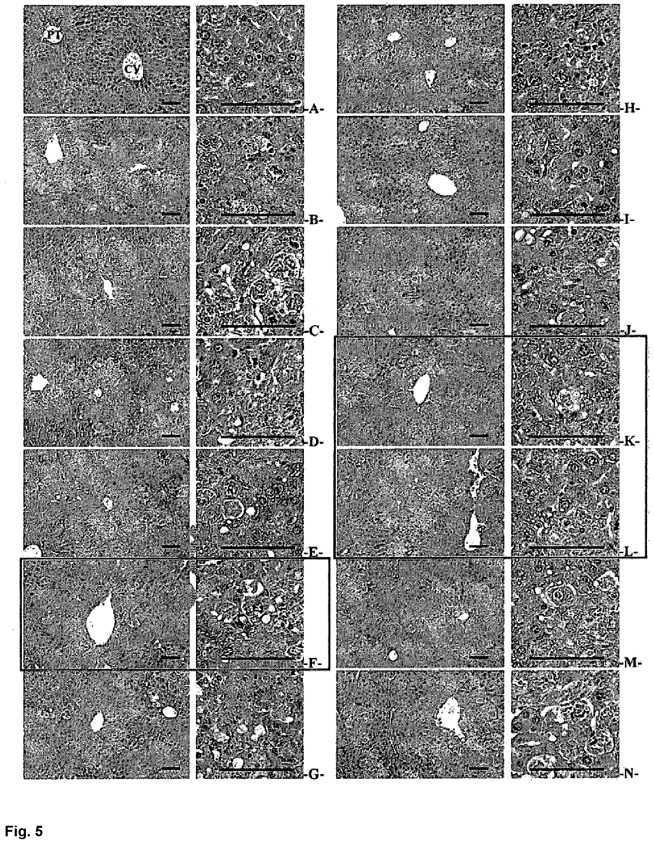

In Figs. 2 and 5, there are provided:
**A** = Normal control (mice treated with distilled water and olive oil);
**B** = CCl₄ control (mice treated with distilled water and CCl₄ 0.5 ml/kg);
**C** = Comparison (mice treated with silymarin 100 mg/kg and CCl₄ 0.5 ml/kg);
**D** = LB (mice treated with LB single composition 200 mg/kg and CCl₄ 0.5 ml/kg);
**E** = DL (mice treated with DL single composition 200 mg/kg and CCl₄ 0.5 ml/kg);
**F** = LB:DL = 1:1 (mice treated with mixture of LB:DL = 1:1 200 mg/kg and CCl₄ 0.5 ml/kg);
**G** = LB:DL = 1:2 (mice treated with mixture of LB:DL = 1:2 200 mg/kg and CCl₄ 0.5 ml/kg);
**H** = LB:DL = 1:4 (mice treated with mixture of LB:DL = 1:4 200 mg/kg and CCl₄ 0.5 ml/kg);
**I** = LB:DL = 1:6 (mice treated with mixture of LB:DL = 1:6 200 mg/kg and CCl₄ 0.5 ml/kg);
**J** = LB:DL = 1:8 (mice treated with mixture of LB:DL = 1:8 200 mg/kg and CCl₄ 0.5 ml/kg);
**K** = LB:DL = 2:1 (mice treated with mixture of LB:DL = 2:1 200 mg/kg and CCl₄ 0.5 ml/kg);
**L** = LB:DL = 4:1 (mice treated with mixture of LB:DL = 4:1 200 mg/kg and CCl₄ 0.5 ml/kg);
**M** = LB:DL = 6:1 (mice treated with mixture of LB:DL = 6:1 200 mg/kg and CCl₄ 0.5 ml/kg); and
**N** = LB:DL = 8:1 (mice treated with mixture of LB:DL = 8:1 200 mg/kg and CCl₄ 0.5 ml/kg).

In Fig. 3, there are provided:
^{a}p<0.01 compared to the normal control according to an LSD test;
^{b}p<0.01 compared to the CCl₄ control according to the LSD test;
^{c}p<0.01 and ^{d}p<0.05 compared to the LB single composition according to the LSD test;
^{e}p<0.01 compared to the DL single composition according to the LSD test;
^{f}p<0.01 compared to the normal control according to the MWtest;
^{g}p<0.01 compared to the CCl₄ control according to the MW test;
^{h}p<0.01 compared to the LB single composition according to the MWtest; and
ⁱp<0.01 compared to the DL single composition according to the MWtest.

With regard to the absolute liver weights, the CCl₄ control group showed a change of 49.40% compared to the normal vehicle control group. However, the groups dosed with silymarin 100 mg/kg, LB and DL single compositions 200 mg/kg, and the mixed compositions of LB:DL = 1:1, 1:2, 1:4, 1:6, 1:8, 2:1, 4:1, 6:1 and 8:1 200 mg/kg showed a change of -14.85, -17.48, -16.70, -21.29, -18.48, -17.54, -17.14, -17.35, -24.92, -22.90, -17.04 and -16.90% compared to the CCl₄ control group, respectively.

With regard to the relative liver weights, the CCl₄ control group showed a change of 68.95% compared to the normal vehicle control group. However, the groups dosed with silymarin 100 mg/kg, LB and DL single compositions 200 mg/kg, and the mixed compositions of LB:DL = 1:1, 1:2, 1:4, 1:6, 1 :8, 2:1, 4:1, 6:1 and 8:1 200 mg/kg showed a change of -20.89, -23.06, -22.15, -28.96, -25.06, -23.91, -23.80, -22.84, -32.40, -30.22, -23.79 and -23.59% compared to the CCl₄ control group, respectively.

### 3. Change in AST and ALT contents in blood

In the CCl₄ control group, a significant increase in AST and ALT contents in blood (p<0.01) was identified respectively compared to the normal vehicle control group. However, all the groups dosed with experimental substances including the DL single composition 200 mg/kg showed a significant decrease in AST and ALT contents in blood (p<0.01) compared to the CCl₄ control group. In particular, in the groups dosed with the mixed compositions of LB:DL = 2:1, 4:1 and 1:1, a significant decrease in AST and ALT contents in blood (p<0.01 or p<0.05) was identified in the same order compared to the group dosed with each of LB and DL single compositions (Fig. 4).

In Fig. 4, there are provided:
^{a}p<0.01 compared to the normal control according to the LSD test;
^{b}p<0.01 compared to the CCl₄ control according to the LSD test;
^{c}p<0.01 and ^{d}p<0.05 compared to the LB single composition according to the LSD test;
^{e}p<0.01 compared to the DL single composition according to the LSD test;
^{f}p<0.01 and ^{g}p<0.05 compared to the normal control according to the MWtest;
^{h}p<0.01 compared to the CCl₄ control according to the MWtest;
ⁱp<0.01 compared to the LB single composition according to the MWtest; and
^{j}p<0.01 compared to the DL single composition according to the MWtest.

With regard to the AST content in blood, the CCl₄ control group showed a change of 286.63% compared to the normal vehicle control group. However, the groups dosed with silymarin 100 mg/kg, LB and DL single compositions 200 mg/kg, and the mixed compositions of LB:DL = 1:1, 1:2, 1:4, 1:6, 1:8, 2:1, 4:1, 6:1 and 8:1 200 mg/kg showed a change of -36.74, -45.92, -41.93, -53.69, -45.41, -44.25, -44.03, -43.05, -66.35, -58.71, -46.74 and -44.68% compared to the CCl₄ control group, respectively.

With regard to the ALT content in blood, the CCl₄ control group showed a change of 561.60% compared to the normal vehicle control group. However, the groups dosed with silymarin 100 mg/kg, LB and DL single compositions 200 mg/kg, and the mixed compositions of LB:DL = 1:1, 1:2, 1:4, 1:6, 1:8, 2:1, 4:1, 6:1 and 8:1 200 mg/kg showed a change of -27.59, -49.54, -44.48, -58.39, -49.02, -47.82, -45.40, -44.89, -66.26, -60.63, -50.57 and -50.23% compared to the CCl₄ control group, respectively.

### 4. Change in lipid peroxidation in liver tissues

In the CCl₄ control group, a significant increase in MDA content in liver tissues (p<0.01), i.e., an increase in lipid peroxidation was identified compared to the normal vehicle control group. However, all the groups dosed with experimental substances including the mixed composition of LB:DL = 1:1 200 mg/kg showed a significant decrease in MDA content in liver tissues (p<0.01) compared to the CCl₄ control group. In particular, in the groups dosed with the mixed compositions of LB:DL = 2:1, 4:1 and 1:1, a significant decrease in lipid peroxidation in liver tissues (p<0.01 or p<0.05) was identified in the same order compared to the group dosed with each of LB and DL single compositions (Fig. 4).

**Table 4**

| Group | | Lipid peroxidation (MDA nM/protein mg) | GSH content (nM/mg protein) | Enzyme activity | |
|---|---|---|---|---|---|
| | | | | SOD (U/mg protein) | CAT (U/mg protein) |
| Control group | Normal | 1.74±0.96 | 47.56±13.09 | 467.54±88.71 | 259.02±81.43 |
| | CCl₄ | 8.88±1.62^{a} | 3.73±1.34^{g} | 48.35±21.51^{g} | 47.46±10.51^{g} |
| Comparative group | Silymarin | 6.03±0.91^{ac} | 17.76±2.70^{gl} | 173.39±34.64^{gl} | 132.93±27.55^{gl} |
| | LB single | 4.72±0.56^{ac} | 26.98±4.44^{gl} | 227.20±35.57^{gl} | 166.33±21,39^{gl} |
| | DL single | 5.29±0.83^{ac} | 22.56±6.59^{gl} | 204.30±27.49^{gl} | 140.33±37.26^{gl} |
| Experimental group [LB:DL] | 1:1 | 3.68±0.43^{acef} | 33.69±5.26^{hikl} | 282.36±31.64^{gijl} | 192.20±14.75^{hikm} |
| | 1:2 | 4.88±0.81^{ac} | 27.84±6.18^{gl} | 225.81±31.38^{gl} | 162.55±19.84^{gl} |
| | 1:4 | 5.10±1.12^{ac} | 26.82±4.27^{gl} | 217.17±34.70^{gl} | 161.88±37.03^{gl} |
| | 1:6 | 5.45±1.28^{ac} | 26.78±3.27^{gl} | 208.31±40.47^{gl} | 153.01±49.17^{gl} |
| | 1:8 | 5.56±1.39^{ac} | 25.81 ±6.38^{gl} | 205.05±21.08^{gl} | 148.73±33.97^{gl} |
| | 2:1 | 2.81±0.38^{bcdf} | 37.71±5.66^{ijl} | 361,83±35.60^{gijl} | 251,16±43.93^{ijm} |
| | 4:1 | 3.58±0.62^{acef} | 35.94±5.48^{ijl} | 328.29±63.76^{gijl} | 209.65±21.07^{ijm} |
| | 6:1 | 4.80±0.77^{ac} | 27.30±6.82^{gl} | 232.49±42.82^{gl} | 170.81±47.19^{gl} |
| | 8:1 | 4.66±1.12^{ac} | 26.28±3.72^{gl} | 230.25±58.84^{gl} | 166.51±38.29^{gl} |
| The values are indicated as mean±SD of eight individual mice (unit: g). ^{a}p<0.01 and ^{b}p<0.05 compared to the normal control according to the LSD test; ^{c}p<0.01 compared to the CCl₄ control according to the LSD test; ^{d}p<0.01 and ^{e}p<0.05 compared to the LB single composition according to the LSD test; ^{f}p<0.01 compared to the DL single composition according to the LSD test; ^{g}p<0.01 and ^{h}p<0.05 compared to the normal control according to the MW test; and ⁱp<0.01 compared to the CCl₄ control according to the MW test; ^{j}p<0.01 and ^{k}p<0.05 compared to the LB single composition according to the MW test; and ^{l}p<0.01 and ^{m}p<0.05 compared to the DL single composition according to the MW test. | | | | | |

With regard to the lipid peroxidation in liver tissues, the CCl₄ control group showed a change of 510% compared to the normal vehicle control group. However, the groups dosed with silymarin 100 mg/kg, LB and DL single compositions 200 mg/kg, and the mixed compositions of LB:DL = 1:1, 1:2, 1:4, 1:6, 1:8, 2:1, 4:1, 6:1 and 8:1 200 mg/kg showed a change of -32.10, -46.83, -40.50, -58.53, -45.03, -42.58, -38.61, -37.41, -68.33, -59.73, -46.00 and -47.59% compared to the CCl₄ control group, respectively.

### 5. Change in GSH content in liver tissues

In the CCl₄ control group, a significant decrease in GSH content, i.e., an endogenous antioxidant in liver tissues (p<0.01) was identified compared to the normal vehicle control group. However, all the groups dosed with experimental substances including the mixed composition of LB:DL = 1:2 200 mg/kg showed a significant increase in GSH content in liver tissues (p<0.01) compared to the CCl₄ control group. In particular, in the groups dosed with the mixed compositions of LB:DL = 2:1, 4:1 and 1:1, a significant increase in GSH content in liver tissues (p<0.01 or p<0.05) was identified in the same order compared to the group dosed with each of LB and DL single compositions (Fig. 4).

With regard to the GSH content in liver tissues, the CCl₄ control group showed a change of -92.16% compared to the normal vehicle control group. However, the groups dosed with silymarin 100 mg/kg, LB and DL single compositions 200 mg/kg, and the mixed compositions of LB:DL = 1:1, 1:2, 1:4, 1:6, 1:8, 2:1, 4:1, 6:1 and 8:1 200 mg/kg showed a change of 376.43, 623.88, 505.26, 803.89, 646.78, 619.38, 618.38, 592.42, 911.64, 864.29, 632.33 and 605.13% compared to the CCl₄ control group, respectively.

### 6. Change in SOD activity in liver tissues

In the CCl₄ control group, a significant decrease in CAT activity, i.e., an endogenous antioxidant enzyme in liver tissues (p<0.01) was identified compared to the normal vehicle control group. However, all the groups dosed with experimental substances including the mixed composition of LB:DL = 1:4 200 mg/kg showed a significant increase in CAT activity in liver tissues (p<0.01) compared to the CCl₄ control group. In particular, in the groups dosed with the mixed compositions of LB:DL = 2:1, 4:1 and 1:1, a significant increase in CAT activity in liver tissues (p<0.01) was identified in the same order compared to the group dosed with each of LB and DL single compositions (Fig. 4).

With regard to SOD activity in liver tissues, the CCl₄ control group showed a change of -89.66% compared to the normal vehicle control group. However, the groups dosed with silymarin 100 mg/kg, LB and DL single compositions 200 mg/kg, and the mixed compositions of LB:DL = 1:1, 1:2, 1:4, 1:6, 1:8, 2:1, 4:1, 6:1 and 8:1 200 mg/kg showed a change of 258.64, 369.96, 322.59, 484.05, 367.09, 349.20, 330.88, 324.15, 648.44, 579.05, 380.90 and 376.26% compared to the CCl₄ control group, respectively.

### 7. Change in CAT activity in liver tissues

In the CCl₄ control group, a significant decrease in CAT activity, i.e., an endogenous antioxidant enzyme in liver tissues (p<0.01) was identified compared to the normal vehicle control group. However, all the groups dosed with experimental substances including the mixed composition of LB:DL = 1:6 200 mg/kg showed a significant increase in CAT activity in liver tissues (p<0.01) compared to the CCl₄ control group. In particular, in the groups dosed with the mixed compositions of LB:DL = 2:1, 4:1 and 1:1, a significant increase in CAT activity in liver tissues (p<0.01 or p<0.05) was identified in the same order compared to the group dosed with each of LB and DL single compositions (Fig. 4).

With regard to CAT activity in liver tissues, the CCl₄ control group showed a change of -81.68% compared to the normal vehicle control group. However, the groups dosed with silymarin 100 mg/kg, LB and DL single compositions 200 mg/kg, and the mixed compositions of LB:DL = 1:1, 1:2, 1:4, 1:6, 1:8, 2:1, 4:1, 6:1 and 8:1 200 mg/kg showed a change of 180.10, 250.48, 195.70, 304.99, 242.72, 241.10, 222.41, 213.39, 429.23, 341.75, 259.93 and 250.86% compared to the CCl₄ control group, respectively.

### 8. Histopathological changes

In the CCl₄ control group, the findings of centrilobular necrosis such as the vacuolation of liver cells, the accumulation of fat droplets in liver cells and the infiltration of inflammatory cells were observed and thus a significant increase in a rate of liver degeneration, the number of degenerated liver cells and the number of infiltrating inflammatory cells as well as an increase in HAI scores related thereto were identified respectively compared to the normal vehicle control group. However, all the groups dosed with experimental substances including the mixed composition of LB:DL = 1:8 200 mg/kg showed a significant decrease in the findings of CCl₄-induced centrilobular necrosis (p<0.01) compared to the CCl₄ control group. In particular, in the groups dosed with the mixed compositions of LB:DL = 2:1, 4:1 and 1:1, a significant decrease in the rate of liver degeneration, the number of degenerated liver cells, the number of infiltrating inflammatory cells in liver parenchyma and HAI scores (p<0.01) was identified in the same order compared to the group dosed with each of LB and DL single compositions (Table 5 and Fig. 5).

**Table 5**

| Group | | HAI (max. = 10) | Area of degraded regions (%/mm²) | Number of DE liver cells (cells/1000 liver cells) | Number of infiltrating inflammatory cells (cells/mm²) |
|---|---|---|---|---|---|
| Control group | Normal | 0.25±0.46 | 2.77±1.96 | 24.88±15.02 | 21.13±10.97 |
| | CCl₄ | 8.50±1.07^{a} | 77.90±10.20^{f} | 765.13±120.37^{f} | 204.75±52.48^{f} |
| Comparative group | Silymarin | 5.00±1.20^{ac} | 51.64±10.41^{fg} | 508.88±107.33^{fg} | 91.63±12.73^{fg} |
| | LB single | 4.13±0.83^{ac} | 44.76±10.08^{fg} | 438.00±96.03^{fg} | 70.00±12.65^{fg} |
| | DL single | 4.50±0.93^{ac} | 50.38±10.20^{fg} | 482.75±112.57^{fg} | 80.13±18.38^{fg} |
| Experimental group [LB:DL] | 1:1 | 2.50±0.93^{acde} | 31.27±6.26^{fgi} | 295.75±66.28^{fghi} | 48.63±10.91^{fghi} |
| | 1:2 | 4.00±1.07^{ac} | 45.28±10.18^{fg} | 448.63±106.92^{fg} | 72.75±12.74^{fg} |
| | 1:4 | 4.25±1.39^{ac} | 46.65±10.31^{fg} | 455.00±122.44^{fg} | 76.13±12.31^{fg} |
| | 1:6 | 4.38±1.19^{ac} | 47.85±11.15^{fg} | 474.25±118.03^{fg} | 75.88±14.26^{fg} |
| | 1:8 | 4.50±1.20^{ac} | 48.26±10.46^{fg} | 488.38±126.58^{fg} | 77.13±10.67^{fg} |
| | 2:1 | 1.50±0.53^{bcde} | 21.33±4.17^{fghl} | 194.13±49.75^{fghl} | 33.63±11.44^{ghl} |
| | 4:1 | 2.13±0.64^{acde} | 27.89±6.30^{fghl} | 260.63±62.13^{fghl} | 43.88±12.19^{fghl} |
| | 6:1 | 3.75±1.49^{ac} | 41.71±15.54^{fg} | 410.25±169.45^{fg} | 67.50±15.33^{fg} |
| | 8:1 | 3.63±1.41^{ac} | 43.57±12.47^{fg} | 407.88±137.03^{fg} | 69.25±16.85^{fg} |
| The values are indicated as mean±SD of eight individual mice (unit: g). ^{a}p<0.01 and ^{b}p<0.05 compared to the normal control according to the LSD test; ^{c}p<0.01 compared to the CCl₄ control according to the LSD test; ^{d}p<0.01 compared to the LB single composition according to the LSD test; ^{e}p<0.01 compared to the DL single composition according to the LSD test; ^{f}p<0.01 compared to the normal control according to the MW test; and ^{g}p<0.01 compared to the CCl₄ control according to the MW test; ^{h}p<0.01 compared to the LB single composition according to the MW test; and ⁱp<0.01 compared to the DL single composition according to the MWtest. | | | | | |

In Fig. 5, there are provided:
With regard to the rate of degenerated regions in liver parenchyma, the CCl₄ control group showed a change of 2713.50% compared to the normal vehicle control group. However, the groups dosed with silymarin 100 mg/kg, LB and DL single compositions 200 mg/kg, and the mixed compositions of LB:DL = 1:1, 1:2, 1:4, 1:6, 1:8, 2:1, 4:1, 6:1 and 8:1 200 mg/kg showed a change of -33.71, -42.54, -35.33, - 59.86, -41.87, -40.11, -38.57, -38.05, -72.62, -64.20, -46.46 and -44.07% compared to the CCl₄ control group, respectively.

With regard to the number of degenerated liver cells, the CCl₄ control group showed a change of 2975.88% compared to the normal vehicle control group. However, the groups dosed with silymarin 100 mg/kg, LB and DL single compositions 200 mg/kg, and the mixed compositions of LB:DL = 1:1, 1:2, 1:4, 1:6, 1:8, 2:1, 4:1, 6:1 and 8:1 200 mg/kg showed a change of -33.49, -42.75, -36.91, -61.35, - 41.37, -40.53, -38.02, -36.17, -74.63, -65.94, -46.38 and -46.69% compared to the CCl₄ control group, respectively.

With regard to the number of infiltrating inflammatory cells, the CCl₄ control group showed a change of 869.23% compared to the normal vehicle control group. However, the groups dosed with silymarin 100 mg/kg, LB and DL single compositions 200 mg/kg, and the mixed compositions of LB:DL = 1:1, 1:2, 1:4, 1:6, 1:8, 2:1, 4:1, 6:1 and 8:1 200 mg/kg showed a change of -55.25, -65.81, -60.87, - 76.25, -64.47, -62.82, -62.94, -62.33, -83.58, -78.57, -67.03 and -66.18% compared to the CCl₄ control group, respectively.

With regard to the HAI grading scores, the CCl₄ control group showed a change of 3300.00% compared to the normal vehicle control group. However, the groups dosed with silymarin 100 mg/kg, LB and DL single compositions 200 mg/kg, and the mixed compositions of LB:DL = 1:1, 1:2, 1:4, 1:6, 1:8, 2:1, 4:1, 6:1 and 8:1 200 mg/kg showed a change of -41.18, -51.47, -47.06, -70.59, -52.94, -50.00, -48.53, -47.06, -82.35, -75.00, -55.88 and -57.35% compared to the CCl₄ control group, respectively.

As liver diseases occur with the depletion of an antioxidant defense system caused by a chemical organic solvent, i.e., CCI4, an experimental animal with CCl₄-induced liver damage has been now frequently used in developing a liver protective agent and conducting a pathogenetic study on liver diseases. As a physiological importance of the liver has been well-known so far, the development of liver protective drugs has been continuously in progress. However, with a demand for developing drugs with a less side effect and a powerful liver protective effect, an exploration into the liver protective drugs using natural products has been still in active progress. Thus, in an effort to develop a more effective mixed agent derived from natural products for improving liver functions, the present inventors, etc., have made a comparative evaluation on the liver protective effect of various dandelion extracts and lemon balm extracts, which have been already well-known for their liver protective effects, by using the most common experimental animal model with liver damage, i.e., a mouse model with CCl₄-induced acute liver damage, and thus have identified that LB and DL 200 mg/kg has a relatively more excellent liver protective effect based on an antioxidant activity than silymarin 100 mg/kg and then have selected the LB and DL as a candidate substance for mixed functional foods derived from natural products for improving liver functions. In the present invention, the present inventors tried to select an optimal rate of LB and DL mixed compositions to show an effect of improving liver function by using the mouse model with CCl₄-induced acute liver damage, too. In other words, LB and DL single compositions and the nine species of LB and DL mixed compositions (at a ratio of LB:DL = 1:1, 1:2, 1:4, 1:6, 1:8, 2:1, 4:1, 6:1 and 8:1, g/g) were respectively dissolved in sterile distilled water, and then orally administered once a day for seven days at a dose of 10 ml/g (200 mg/kg), after which CCl₄ was intraperitoneally administered once at 0.5 ml/kg in one hour after a last seventh oral administration to induce an acute liver damage. In 24 hours after CCl₄ administration, observation was made along with findings in gross liver autopsy, liver weights, AST and ALT contents in blood, lipid peroxidation of liver antioxidant defense system, GSH content and histopathological change in liver tissues with SOD and CAT activities, and a change in weights and weight gains was also observed for the whole experimental period of seven days. Histopathologically, a degree of liver damage was evaluated based on HAI grading scores, a rate of degenerated regions in liver parenchyma (%/mm²), the number of degenerated liver cells (cells/1000 liver cells) and the number of infiltrating inflammatory cells (cells/mm²). In the present experiment, an LB:DL mixed composition was considered to simultaneously show a synergy effect with a statistically significant rise in a medicinal efficacy (p<0.01 or p<0.05) compared to each of LB and DL single compositions. Further, a group orally dosed with silymarin 100 mg/kg, which is a liver protective agent derived from natural products and has been already well-known to have a liver protective effect based on its antioxidant effect, was used as a control drug group.

As a result of the present experiment, the findings of typical centrilobular acute liver damage caused by oxidative stress were identified due to a single intraperitoneal administration of CCl₄ 0.5 ml/kg, that is, a remarkable decrease in weights and weight gains, an increase in liver weights by gross liver nodule formation and hepatomegaly, an increase in AST and ALT contents in blood, an increase in liver lipid peroxidation, depletion of endogenous antioxidant (GSH) and antioxidant enzymes (SOD and CAT), and an increase in modified HAI scores by centrilobular necrosis. Meanwhile, due to an oral administration of all the LB and DL single compositions 200 mg/kg and the nine species of mixed compositions of LB:DL = 1:1, 1:2, 1:4, 1:6, 1:8, 2:1, 4:1, 6:1 and 8:1 (g/g) 200 mg/kg, such findings of centrilobular acute liver damage caused by CCl₄-induced oxidative stress showed a consistently remarkable decrease in a more excellent degree than the group dosed with silymarin 100 mg/kg. In particular, the groups dosed with the mixed compositions of LB:DL = 2:1, 4:1 and 1:1 showed the findings of inhibiting of a significant decrease in weights and weight gains by CCl₄, an increase in liver weights by gross liver nodule formation and hepatomegaly, an increase in AST and ALT contents in blood, an increase in liver lipid peroxidation, depletion of endogenous antioxidant (GSH) and antioxidant enzymes (SOD and CAT), and increase in modified HAI scores by centrilobular necrosis in the same order. Thus, at least under the conditions of the present experiment, it was determined that the mixed compositions of LB:DL = 2:1, 4:1 and 1:1 (g/g) increase a liver protective effect in a more synergistic way through the antioxidant defense system of LB and DL in the same order. Out of those compositions, it was determined that the mixed composition of LB:DL = 2:1 (g/ g) showing the most excellent effect would have an especially high chance to be developed as a novel and more effective mixed agent derived from natural products for improving liver functions.

A decrease in weights by CCl₄ administration is known to be caused not only by the intrinsic toxicity of CCl₄ but also occurs as a secondary result from liver damage, and thus the inhibiting of weight decrease by CCl₄ has been used as an evidence for indirectly determining if a candidate substance has the liver protective effect. The mice of the normal vehicle control group used in the present experiment showed an increase in weights respectively within a range of weight gains of the age-matched normal ICR mice. However, the CCl₄ control group showed a significant decrease in weights compared to the normal vehicle control group and such decrease was identified exclusively on a final day of sacrifice in 24 hours after CCl₄ administration. In this regard, the former group also showed a significant decrease in weight gains for seven days compared to the normal vehicle control group. Meanwhile, in all the groups dosed with the experimental substances including silymarin 100 mg/kg, a significant increase in weights was identified on a final day of sacrifice in 24 hours after CCl₄ administration compared to the CCl₄ control group, and a significant increase in weight gains for seven days was also shown compared to the CCl₄ control group. In particular, in the groups dosed with LB and DL single compositions 200 mg/kg and all the nine species of LB:DL mixed compositions 200 mg/kg, a more excellent effect on inhibiting CCl₄-induced decrease in weights was identified compared to silymarin 100 mg/kg. In the groups dosed with the mixed compositions of LB:DL = 2:1, 4:1 and 1:1, a significant increase in weight gains was identified in the same order compared to each of the groups dosed with LB and DL single compositions. At least under the conditions of the present experiment, such results are determined as a direct evidence by which the mixed compositions of LB:DL = 2:1, 4:1 and 1:1 (g/g) increase an effect of LB and DL on inhibiting CCl₄-induced decrease in weights in a more synergic way.

The CCl₄ administration causes an increase in liver weights due to gross liver nodule formation and hepatomegaly. As a result of the present experiment, in the CCl₄ control group, a remarkable increase in liver weights was also identified respectively due to gross liver nodule formation and hepatomegaly compared to the normal vehicle control group. Meanwhile, all the groups dosed with the experimental substances including LB single composition 200 mg/kg showed a significant decrease in absolute and relative liver weights and a remarkable decrease in findings of hepatomegaly accompanied by nodule formation compared to the CCl₄ control group. In particular, in the groups dosed with LB and DL single compositions 200 mg/kg and all the nine species of LB:DL mixed compositions 200 mg/kg, a more excellent effect of inhibiting a CCl₄-induced increase in absolute and relative liver weights and the findings of hepatomegaly accompanied by nodule formation was identified compared to silymarin 100 mg/kg. In the groups dosed with the mixed compositions of LB:DL = 2:1, 4:1 and 1:1, a significant decrease in absolute and relative liver weights and a remarkable decrease in the findings of hepatomegaly accompanied by nodule formation were also identified in the same order compared to each of the groups dosed with LB and DL single compositions. At least under the conditions of the present experiment, such results are determined as one of the indirect evidences by which the mixed compositions of LB:DL = 2:1, 4:1 and 1:1 (g/g) increase an effect of LB and DL on protecting a CCl₄-induced acute liver damage in a more synergic way in the same order.

In general, AST and ALT have been used as a representative blood biochemical marker for determining a liver damage. Even the CCl₄-induced liver damage has been also accompanied by a remarkable increase in the AST and ALT contents in blood. From the results of the present experiment, a remarkable increase in the AST and ALT contents in blood was identified respectively in the CCl₄ control group. Meanwhile, all the groups dosed with the experimental substances including DL single composition 200 mg/kg showed a significant decrease in the AST and ALT contents in blood compared to the CCl₄ control group. In particular, in the groups dosed with LB and DL single compositions 200 mg/kg and all the nine species of LB:DL mixed compositions 200 mg/kg, a more excellent effect of inhibiting a CCl₄-induced increase in the AST and ALT contents in blood was identified compared to silymarin 100 mg/kg. In the groups dosed with the mixed compositions of LB:DL= 2:1, 4:1 and 1:1, a significant decrease in the AST and ALT contents in blood was also identified in the same order compared to each of the groups dosed with LB and DL single compositions. At least under the conditions of the present experiment, such results are determined as another reliable evidence by which the mixed compositions of LB:DL = 2:1, 4:1 and 1:1 (g/g) increase an effect of LB and DL on protecting a CCl₄-induced acute liver damage in a more synergic way in the same order.

Through various clinical and experimental studies, oxidative stress has been highly considered as a cause of CCl₄-induced liver damage. Lipid peroxidation is an autocatalytic reaction which causes an oxidative disruption of cell membranes. The disruption of cell membranes by lipid peroxidation promotes the formation of noxious reactive aldehyde metabolites, that is, free radicals, which are classified into MDA, along with apoptosis. The various reactive oxygen species (ROS) formed as above cause the oxidative damage of various biological giant molecules including lipid, protein and DNA, and oxidative stress has an influence on body fat cells and causes a decrease in weights along with a decrease in fat mass, too. MDA, a final product of lipid peroxidation, has been usefully used as an indicator for measuring the lipid peroxidation. An experimental animal model with CCl₄-induced liver damage also showed a remarkable increase in MDA content in liver tissues, that is, causing the lipid peroxidation. From the results of the present experiment, it was identified that the CCl₄ control group shows a remarkable increase in MDA content. GSH, a kind of representative endogenous antioxidants, is known to control a damage to tissues by removing the ROS at a relatively low concentration from cells. Further, SOD, an endogenous antioxidant enzyme, serves as a part of an enzyme defense system of cells and CAT is also a representative endogenous antioxidant enzyme which converts noxious hydrogen peroxide (H₂O₂) into harmless water (H₂O). Now, a decrease in the endogenous antioxidant GSH along with less activities of antioxidant enzymes SOD and CAT in tissues means a failure of compensatory action by CCl₄-induced oxidative stress. As a result of the present experiment, a remarkable decrease in liver lipid peroxidation and an increase in active GSH content and SOD and CAT activities of an antioxidant defense system were identified in all the groups dosed with experimental substances including the mixed composition of LB:DL = 1:2 compared to the CCl₄ control group. In particular, in the groups dosed with LB and DL single compositions 200 mg/kg and all the nine species of LB:DL mixed compositions 200 mg/kg, a more excellent effect of inhibiting a CCl₄-induced liver lipid peroxidation and a decrease in the antioxidant defense system was identified compared to silymarin 100 mg/kg. In the groups dosed with the mixed compositions of LB:DL = 2:1, 4:1 and 1:1, a significant inhibition of liver lipid peroxidation and an increase in the activity of the antioxidant defense system were identified in the same order compared to the groups dosed with each of LB and DL single compositions. At least under the conditions of the present experiment, such results are determined as a clear evidence by which the mixed compositions of LB:DL = 2:1, 4:1 and 1:1 (g/g) increase an effect of LB and DL on protecting a CCl₄-induced liver damage based on an antioxidant activity in a more synergic way in the same order.

As a result of the present experiment, histopathologically the vacuolation (accumulation of fat droplets) and ballooning of liver cells and the infiltration of inflammatory cells were identified as centrilobular due to a single intraperitoneal administration of CCl₄ 0.5 ml/kg, and a CCl₄-induced histopathological liver damage was identified again by using a histomorphological method for a modified HAI scoring system, a rate of liver degeneration, the number of degenerated liver cells and the number of infiltrating inflammatory cells. In other words, in the CCl₄ control group, a significant increase in the rate of liver degeneration, the number of degenerated liver cells and the number of infiltrating inflammatory cells as well as an increase in HAI scores related thereto were identified respectively compared to the normal vehicle control group. However, all the groups dosed with experimental substances including the mixed composition of LB:DL = 1:8 200 mg/kg showed a significant decrease in the findings of CCl₄-induced centrilobular necrosis (p<0.01) compared to the CCl₄ control group. In particular, in the groups dosed with LB and DL single compositions 200 mg/kg and all the nine species of LB:DL mixed compositions 200 mg/kg, a more excellent effect of inhibiting the CCl₄-induced liver lipid peroxidation and a decrease in the antioxidant defense system was identified compared to each of LB and DL single compositions. In the groups dosed with the mixed compositions of LB:DL = 2:1, 4:1 and 1:1, a significant decrease in the rate of liver degeneration, the number of degenerated liver cells, the number of infiltrating inflammatory cells in liver parenchyma and HAI scores was identified in the same order compared to the group dosed with each of LB and DL single compositions. At least under the conditions of the present experiment, such histopathological results are determined as a clear and direct evidence by which the mixed compositions of LB:DL = 2:1, 4:1 and 1:1 (g/g) increase an effect of LB and DL on inhibiting CCl₄-induced centrilobular necrosis in a more synergic way in the same order.

From the results above, at least under the conditions of the present experiment, the findings of centrilobular acute liver damage caused by CCl₄-induced oxidative stress, a CCl₄-induced decrease in weights and weight gains, an increase in liver weights by gross liver nodule formation and hepatomegaly, an increase in AST and ALT contents in blood, an increase in liver lipid peroxidation, depletion of endogenous antioxidant (GSH) and antioxidant enzymes (SOD and CAT), and an increase in modified HAI scores by centrilobular necrosis showed a consistently remarkable decrease due to an oral administration of all the LB and DL single compositions 200 mg/kg and the nine species of mixed compositions of LB:DL = 1:1, 1:2, 1:4, 1:6, 1:8, 2:1, 4:1, 6:1 and 8:1 (g/g) 200 mg/kg compared to the group dosed with silymarin 100 mg/kg. In particular, the groups dosed with the **mixed compositions** of LB:DL = 2:1, 4:1 **and 1:1 showed the findings of significantly inhibiting of centrilobular acute liver damage caused by CCl₄-induced oxidative stress compared to the group dosed with each of LB and DL single compositions in the same order.** Thus, it was determined that the **mixed compositions** of LB:DL = 2:1, 4:1 and **1:1 (g/g) increase a liver protective effect of LB and DL based on an antioxidant defense system in a more synergic way in the same order. Out of them, it is expected that the mixed composition of LB:DL = 2:1 (g/g) showing the most excellent effect is highly likely to be developed as a novel and more effective mixed agent derived from natural products for improving liver functions.**

### Preparation Example 3: Preparation of imported lemon balm leaf extract powder

A brown-colored extract powder (LB-F) was obtained by carrying out the same process as shown in Preparation Example 1 above with an exception of using imported lemon balm leaves (**France**) instead of home-grown lemon balm leaves.

### Preparation Example 4: Preparation of whole dandelion plant extract powder

A brown-colored extract powder was obtained by carrying out the same process as shown in Preparation Example 2 above with an exception of using imported dandelion leaves (DL-F, **France**), imported dandelion roots (DR-F, **France**) and home-grown dandelion roots (DR-K) instead of home-grown dandelion leaves.

The experiments above were repeated in such a way that the extracts of Preparation Examples 3 and 4 above were compared with silymarin, respectively.

In other words, a liver protective effect of the five species of natural product extract (DL-F, DR-F, DL, DR-K and LB-F) was compared and evaluated by using a mouse model with CCl₄-induced acute liver damage. In other words, the five species of natural product extract were orally administered once daily for seven days at a dose of 200 ml/kg respectively and CCl₄ 0.5 ml/kg was intraperitoneally administered once in one hour after a last seventh oral administration to induce an acute liver damage. In 24 hours after CCl₄ administration, observation was made along with findings in gross liver autopsy, liver weights, AST and ALT contents in blood, lipid peroxidation of liver antioxidant defense system, GSH content and histopathological change in liver tissues with SOD and CAT activities, and a change in weights and weight gains was also observed for the whole experimental period of seven days. Histopathologically, a degree of liver damage was evaluated based on HAI grading scores, a rate of degenerated regions in liver parenchyma (%/mm²), the number of degenerated liver cells (cells/1000 liver cells) and the number of infiltrating inflammatory cells (cells/mm²), as well as a numerical change in cells (cells/1000 liver cells) immunoreactive to an apoptosis marker, i.e., split caspase-3 and PARP, an NO-related oxidative stress marker, i.e., NT, and a lipid peroxidation marker, i.e., 4-HNE among 1000 liver cells through an ABC-based immunohistochemical method. The experimental results were compared with the group dosed with silymarin 100 mg/kg, which is a liver protective agent derived from natural products and has been already well-known to have a liver protective effect based on its antioxidant effect.

A total of 84 individual SPF/VAF Outbred CrljOri:CD1[ICR] male mice (OrientBio, Seungnam, Korea) were acclimated for seven days, then screened and divided into each group of ten individuals based on weights (33.23±1.20 g on average and 30.40 to 35.70 g) measured one day before administering an experimental substance, and then a total of eight groups were used in the experiment. All the experimental animals were fasted (with a free access to drinking water) for 18 hours before starting the administration of experimental substances and before a final day of autopsy.

### Group separation (a total of 6 groups with 10 individuals per group)

Normal vehicle control group: Normal vehicle control group dosed with olive oil after administration of sterile distilled water

CCl₄ control group: Vehicle control group with acute liver damage induced by administration of CCl₄ 0.5 ml/kg after administration of sterile distilled water
Control drug group dosed with silymarin 100 mg/kg and CCl₄ 0.5 ml/kg
Experimental group dosed with DL-F 200 mg/kg and CCl₄ 0.5 ml/kg
Experimental group dosed with DR-F 200 mg/kg and CCl₄ 0.5 ml/kg
Experimental group dosed with DL 200 mg/kg and CCl₄ 0.5 ml/kg
Experimental group dosed with DR-K 200 mg/kg and CCl₄ 0.5 ml/kg
Experimental group dosed with LB-F 200 mg/kg and CCl₄ 0.5 ml/kg

### 1. Change in weights

The CCl₄ control group showed a significant decrease in weights (p<0.01) compared to the normal vehicle control group and such decrease was identified exclusively on a final day of sacrifice in 24 hours after CCl₄ administration. In this regard, the former group also showed a significant decrease in weights (p<0.01) for seven days compared to the normal vehicle control group. Meanwhile, a significant increase in weights (p<0.01 or p<0.05) was identified in an order of LB-F, DL-F, silymarin, DR-F, DR-K and DL on a final day of sacrifice in 24 hours after CCl₄ administration compared to the CCl₄ control group, and a significant increase in weight gains (p<0.01) for seven days was also shown compared to the CCl₄ control group.

With regard to the weight gains for the whole experimental period of seven days, the CCl₄ control group showed a change of -66.34% compared to the normal vehicle control group. However, the groups dosed with silymarin 100 mg/kg, DL-F, DR-F, DL, DR-K and LB-F 200 mg/kg showed a change of 192.31, 213.46, 175.96, 156.73, 164.42 and 248.08% compared to the CCl₄ control group, respectively.

### 2. Findings in gross liver autopsy and change in weights

The CCl₄ control group showed the findings of hepatomegaly accompanied by a remarkable nodule formation compared to the normal vehicle control group and thus a significant increase in the absolute and relative liver weights (p<0.01) was identified respectively compared to the normal vehicle control group. Meanwhile, a significant decrease in the absolute and relative liver weights (p<0.01) and a remarkable decrease in the findings of hepatomegaly accompanied by a nodule formation were identified in an order of LB-F, DL-F, silymarin, DR-F, DL and DR-K compared to the CCl₄ control group, respectively.

With regard to the absolute liver weights, the CCl₄ control group showed a change of 48.89% compared to the normal vehicle control group. However, the groups dosed with silymarin 100 mg/kg, DL-F, DR-F, DL, DR-K and LB-F 200 mg/kg showed a change of -14.02, -15.71, -11.63, -8.79, -6.83 and -18.10% compared to the CCl₄ control group, respectively.

With regard to the relative liver weights, the CCl₄ control group showed a change of 61.36% compared to the normal vehicle control group. However, the groups dosed with silymarin 100 mg/kg, DL-F, DR-F, DL, DR-K and LB-F 200 mg/kg showed a change of -19.37, -21.59, -16.29, -13.30, -12.04 and -24.80% compared to the CCl₄ control group, respectively.

### 3. Change in AST and ALT contents in blood

In the CCl₄ control group, a significant increase in AST and ALT contents in blood (p<0.01) was identified compared to the normal vehicle control group, respectively. Meanwhile, a significant decrease in AST and ALT contents in blood (p<0.01) was identified in an order of LB-F, DL-F, silymarin, DR-F, DR-K and DL compared to the CCl₄ control group, respectively.

With regard to the AST content in blood, the CCl₄ control group showed a change of 341.72% compared to the normal vehicle control group. However, the groups dosed with silymarin 100 mg/kg, DL-F, DR-F, DL, DR-K and LB-F 200 mg/kg showed a change of -37.64, -41.11, -31.63, -23.65, -28.68 and -46.25% compared to the CCl₄ control group, respectively.

With regard to the ALT content in blood, the CCl₄ control group showed a change of 575.38% compared to the normal vehicle control group. However, the groups dosed with silymarin 100 mg/kg, DL-F, DR-F, DL, DR-K and LB-F 200 mg/kg showed a change of -32.21, -41.37, -25.06, -17.18, -20.50 and -51.58% compared to the CCl₄ control group, respectively.

### 4. Change in lipid peroxidation in liver tissues

In the CCl₄ control group, a significant increase in the MDA content in liver tissues (p<0.01), i.e., an increase in lipid peroxidation was identified compared to the normal vehicle control group. However, a significant decrease in MDA content in liver tissues (p<0.01) was identified in an order of LB-F, DL-F, silymarin, DR-F, DR-K and DL compared to the CCl₄ control group, respectively.

With regard to the lipid peroxidation in liver tissues, the CCl₄ control group showed a change of 453.65% compared to the normal vehicle control group. However, the groups dosed with silymarin 100 mg/kg, DL-F, DR-F, DL, DR-K and LB-F 200 mg/kg showed a change of -30.22, -35.80, -26.16, -18.70, -22.78 and -46.13% compared to the CCl₄ control group, respectively.

### 5. Change in GSH content in liver tissues

In the CCl₄ control group, a significant decrease in the GSH content, i.e., an endogenous antioxidant in liver tissues (p<0.01) was identified compared to the normal vehicle control group. However, a significant increase in the GSH content in liver tissues was identified in an order of LB-F, DL-F, silymarin, DR-F, DR-K and DL compared to the CCl₄ control group, respectively.

With regard to the GSH content in liver tissues, the CCl₄ control group showed a change of -92.38% compared to the normal vehicle control group. However, the groups dosed with silymarin 100 mg/kg, DL-F, DR-F, DL, DR-K and LB-F 200 mg/kg showed a change of 466.49, 608.69, 417.64, 212.72, 295.18 and 826.33% compared to the CCl₄ control group, respectively.

### 6. Change in CAT activity in liver tissues

In the CCl₄ control group, a significant decrease in the CAT activity, i.e., an endogenous antioxidant enzyme in liver tissues (p<0.01) was identified compared to the normal vehicle control group. However, a significant increase in the CAT activity in liver tissues (p<0.01) was identified in an order of LB-F, DL-F, silymarin, DR-F, DR-K and DL compared to the CCl₄ control group, respectively.

With regard to the CAT activity in liver tissues, the CCl₄ control group showed a change of -81.72% compared to the normal vehicle control group. However, the groups dosed with silymarin 100 mg/kg, DL-F, DR-F, DL, DR-K and LB-F 200 mg/kg showed a change of 192.51, 226.54, 149.81, 82.26, 137.10 and 259.00% compared to the CCl₄ control group, respectively.

### 7. Change in SOD activity in liver tissues

In the CCl₄ control group, a significant decrease in the SOD activity, i.e., an endogenous antioxidant enzyme in liver tissues (p<0.01) was identified compared to the normal vehicle control group. However, a significant increase in the SOD activity in liver tissues (p<0.01) was identified in an order of LB-F, DL-F, silymarin, DR-F, DR-K and DL compared to the CCl₄ control group, respectively.

With regard to the SOD activity in liver tissues, the CCl₄ control group showed a change of -85.83% compared to the normal vehicle control group. However, the groups dosed with silymarin 100 mg/kg, DL-F, DR-F, DL, DR-K and LB-F 200 mg/kg showed a change of 206.34, 241.01, 174.59, 81.19, 103.50 and 308.91 % compared to the CCl₄ control group, respectively.

### 8. Histopathological changes

In the CCl₄ control group, the findings of centrilobular necrosis such as the vacuolation of liver cells, the accumulation of fat droplets in liver cells and the infiltration of inflammatory cells were observed, and thus a significance increase (p<0.01) in a rate of liver degeneration, the number of degenerated liver cells and the number of infiltrating inflammatory cells, and an increase of HAI scores related thereto were identified compared to the normal vehicle control group, respectively. However, such findings of CCl₄-induced centrilobular necrosis were significantly inhibited in an order of LB-F, DL-F, silymarin, DR-F, DR-K and DL, respectively (p<0.01 or p<0.05).

With regard to the rate of degenerated regions in liver parenchyma, the CCl₄ control group showed a change of 3455.34% compared to the normal vehicle control group. However, the groups dosed with silymarin 100 mg/kg, DL-F, DR-F, DL, DR-K and LB-F 200 mg/kg showed a change of -37.30, -42.03, - 33.71, -17.43, -25.53 and -53.32% compared to the CCl₄ control group, respectively.

With regard to the number of degenerated liver cells, the CCl₄ control group showed a change of 3562.39% compared to the normal vehicle control group. However, the groups dosed with silymarin 100 mg/kg, DL-F, DR-F, DL, DR-K and LB-F 200 mg/kg showed a change of -33.61, -42.41, -33.97, - 16.26, -23.25 and -53.6% compared to the CCl₄ control group, respectively.

sWith regard to the number of infiltrating inflammatory cells, the CCl₄ control group showed a change of 703.17% compared to the normal vehicle control group. However, the groups dosed with silymarin 100 mg/kg, DL-F, DR-F, DL, DR-K and LB-F 200 mg/kg showed a change of -71.50, -73.04, - 62.29, -38.70, -53.64 and -76.88% compared to the CCl₄ control group, respectively.

With regard to the HAI grading score, the CCl₄ control group showed a change of 2866.67% compared to the normal vehicle control group. However, the groups dosed with silymarin 100 mg/kg, DL-F, DR-F, DL, DR-K and LB-F 200 mg/kg showed a change of -49.44, -52.81, -41.57, -29.21, -34.83 and -61.80% compared to the CCl₄ control group, respectively.

### 9. Immunohistochemical observation

In the CCl₄ control group, a significant increase in the number of liver cells (p<0.01) immunoreactive to an apoptosis marker, i.e., split caspase-3 and PARP, an NO-related oxidative stress marker, i.e., NT, and a lipid peroxidation marker, i.e., 4-HNE was identified compared to the normal vehicle control group. However, a significant decrease in the number of liver cells (p<0.01) immunoreactive to split caspase-3 and PARP, NT and 4-HNE was identified in an order of LB-F, DL-F, silymarin, DR-F, DR-K and DL compared to the CCl₄ control group, respectively.

With regard to the number of caspase-3 immunoreactive cells in liver tissues, the CCl₄ control group showed a change of 62808.33% compared to the normal vehicle control group. However, the groups dosed with silymarin 100 mg/kg, DL-F, DR-F, DL, DR-K and LB-F 200 mg/kg showed a change of -48.11, -56.05, -41.01, -33.10, -38.89 and -66.53% compared to the CCl₄ control group, respectively.

With regard to the number of PARP immunoreactive cells in liver tissues, the CCl₄ control group showed a change of 15169.39% compared to the normal vehicle control group. However, the groups dosed with silymarin 100 mg/kg, DL-F, DR-F, DL, DR-K and LB-F 200 mg/kg showed a change of - 61.51, -62.51, -37.02, -20.78, -31.30 and -68.02% compared to the CCl₄ control group, respectively.

With regard to the number of NT immunoreactive cells in liver tissues, the CCl₄ control group showed a change of 2462.89% compared to the normal vehicle control group. However, the groups dosed with silymarin 100 mg/kg, DL-F, DR-F, DL, DR-K and LB-F 200 mg/kg showed a change of - 55.70, -59.01, -37.02, -19.87, -27.57 and -65.70% compared to the CCl₄ control group, respectively.

With regard to the number of 4-HNE immunoreactive cells in liver tissues, the CCl₄ control group showed a change of 4765.16% compared to the normal vehicle control group. However, the groups dosed with silymarin 100 mg/kg, DL-F, DR-F, DL, DR-K and LB-F 200 mg/kg showed a change of - 40.51, -42.43, -37.00, -18.51, -27.34 and -68.59% compared to the CCl₄ control group, respectively.

As a result of the present experiment, the findings of typical centrilobular acute liver damage caused by oxidative stress were identified due to a single intraperitoneal administration of CCl₄ 0.5 ml/kg, that is, a remarkable decrease in weights and weight gains, an increase in liver weights by gross liver nodule formation and hepatomegaly, an increase in AST and ALT contents in blood, an increase in liver lipid peroxidation and depletion of endogenous antioxidant (GSH) and antioxidant enzymes (SOD and CAT), an increase in modified HAI scores by centrilobular necrosis, and an increase in the number of cells immunoreactive to an apoptosis marker, i.e., split caspase-3 and PARP, an NO-related oxidative stress marker, i.e., NT, and a lipid peroxidation marker, i.e., 4-HNE. Meanwhile, such findings of centrilobular acute liver damage caused by CCl₄-induced oxidative stress showed a consistently remarkable decrease in an order of LB-F, DL-F, silymarin, DR-F, DR-K and DL. Thus, at least under the conditions of the present experiment, it was observed that a very excellent protective effect occurs to CCl₄-induced acute liver damage based on an antioxidant defense system in an order of LB-F, LB-F, DL-F, DR-F, DR-K and DL. In particularly, it was observed that LB-F and DL-F 200 mg/kg show a more excellent liver protective effect in a mouse model with CCl₄-induced acute liver damage than silymarin 100 mg/kg, and thus it is determined that LB-F and DL-F would be appropriate as a candidate substance to be developed as a more effective mixed agent derived from natural products for improving liver functions.

The present invention has been described in detail only with regard to the specific embodiments as described above. However, it is apparent to those skilled in the art that the present invention may be variously changed and modified within the technical scope of the present invention and it is also natural that such changes and modifications belong to the accompanying patent claims.

## Claims

1. A composition having an efficacy of improving liver functions, wherein the composition comprises a dandelion extract and a lemon balm extract as effective ingredients.

2. The composition having an efficacy of improving liver functions according to claim 1, wherein the dandelion extract is extracted from whole dandelion plants with a polar solvent selected from the group consisting of water, methanol, ethanol and a mixture of at least two thereof.

3. The composition having an efficacy of improving liver functions according to claim 2, wherein the dandelion extract is extracted from dandelion leaves.

4. The composition having an efficacy of improving liver functions according to claim 1, wherein the lemon balm extract is extracted from whole lemon balm plants with a polar solvent selected from the group consisting of water, methanol, ethanol and a mixture of at least two thereof.

5. The composition having an efficacy of improving liver functions according to claim 4, wherein the lemon balm extract is extracted from lemon balm leaves with a polar solvent selected from the group consisting of water, methanol, ethanol and a mixture of at least two thereof.

6. The composition having an efficacy of improving liver functions according to claim 1, wherein the composition includes the dandelion extract and the lemon balm extract mixed at a weight ratio of 1:8 to 8:1.

7. The composition having an efficacy of improving liver functions according to claim 1, wherein the composition includes the dandelion extract and the lemon balm extract mixed at a weight ratio of 1:1 to 4.

8. A drug having an efficacy of improving liver functions, comprising the composition according to any one of claims 1 to 6 as an effective ingredient.

9. A food having an efficacy of improving liver functions, comprising the composition according to any one of claims 1 to 6 as an effective ingredient.
